Europäisches Patentamt

European Patent Office

Office européen des brevets

(11) Numéro de publication: **0 491 629 B1**

## FASCICULE DE BREVET EUROPEEN

(45) Date de publication de fascicule du brevet: **29.03.95** (51) Int. Cl.6: **A61K 7/06**

(21) Numéro de dépôt: **91403460.8**

(22) Date de dépôt: **19.12.91**

(54) **Laque aérosol pour la fixation des cheveux contenant un tétrapolymère d'acide acrylique, de N-vinylpyrrolidone, de N-tertiobutylachrylamide et de méthacrylate d'éthyle.**

(30) Priorité: **19.12.90 FR 9015929**

(43) Date de publication de la demande:
**24.06.92 Bulletin 92/26**

(45) Mention de la délivrance du brevet:
**29.03.95 Bulletin 95/13**

(84) Etats contractants désignés:
**AT BE CH DE DK ES FR GB GR IT LI NL SE**

(56) Documents cités:
EP-A- 0 256 458     EP-A- 0 379 082
FR-A- 2 424 738     US-A- 3 634 368
US-A- 3 927 199     US-A- 4 859 455

(73) Titulaire: **L'OREAL**
**14, Rue Royale**
**F-75008 Paris (FR)**

(72) Inventeur: **Mondet, Jean**
**34, rue Daniel Fery**
**F-93700 Drancy (FR)**
Inventeur: **Lion, Bertrand**
**2, rue Denis Papin**
**F-93190 Livry Gargan (FR)**

(74) Mandataire: **Stalla-Bourdillon, Bernard et al**
**CABINET NONY & CIE**
**29, rue Cambacérès**
**F-75008 Paris (FR)**

Rank Xerox (UK) Business Services
(3.10/3.09/3.3.3)

**Description**

La présente invention a pour objet une composition cosmétique sous forme d'une laque aérosol pour la fixation des cheveux contenant, en tant que substance filmogène, un tétrapolymère d'acide acrylique, de N-vinylpyrrolidone, de N-tertiobutylacrylamide et de méthacrylate d'éthyle, le gaz propulseur étant du type non halogéné.

Pour des raisons écologiques, la tendance actuelle consiste à remplacer systématiquement, dans les bombes aérosols pour cheveux, les gaz propulseurs halogénés du type Fréon par des hydrocarbures, en particulier par du propane ou du butane ou leurs mélanges. Toutefois une telle substitution ne peut se faire sans modifier profondément les formulations non seulement en ce qui concerne les rapports entre les ingrédients mais également en ce qui concerne la nature même de ceux-ci et tout particulièrement des substances filmogènes.

Il est en effet bien connu qu'une laque aérosol pour la fixation des cheveux doit satisfaire à un certain nombre de critères. Parmi ceux-ci on doit tout particulièrement mentionner un bon pouvoir laquant de la chevelure même en atmosphère à forte teneur en humidité sans constater un effet de poissage de la chevelure.

Enfin, il convient que la chevelure présente un aspect naturel les cheveux étant brillants et souples et qu'ils puissent être peignés sans que l'on constate un effet de poudrage.

Jusqu'à présent les recherches n'ont pas permis de mettre au point de nouveaux polymères présentant une bonne compatibilité avec les propulseurs non halogénés et présentant par ailleurs d'excellentes propriétés cosmétiques.

On peut néanmoins citer la demande de brevet européen 256 458 qui décrit une composition pour la fixation des cheveux contenant, en tant que substance filmogène, un polymère résultant de la polymérisation de :

- 20 à 60 % en poids de N-vinylpyrrolidone,
- 20 à 60 % en poids d'un acrylamide N-mono ou dialkyle substitué, le radical alkyle ayant de 1 à 8 atomes de carbone, et
- 2 à 48 % en poids d'un acrylate ou méthacrylate d'alkyle ou d'hydroxyalkyle, le radical alkyle ayant de 1 à 4 atomes de carbone ou le radical hydroxyalkyle ayant de 2 à 4 atomes de carbone, et
- 3 à 12 % en poids d'acide acrylique ou méthacrylique.

Après différentes études sur ce type de polymères, on a constaté qu'en vue d'obtenir de bonnes propriétés cosmétiques ainsi qu'une bonne compatibilité aux hydrocarbures en solution alcoolique, une sélection très stricte s'imposait non seulement au niveau des monomères mais également de leurs pourcentages respectifs.

On a en effet constaté de façon tout à fait surprenante et inattendue que de très bonnes propriétés cosmétiques et une bonne compatibilité avec les gaz propulseurs non halogénés pouvaient être obtenues grâce à un tétrapolymère contenant des unités d'acide acrylique, de N-vinylpyrrolidone, de N-tertiobutylacrylamide et de méthacrylate d'éthyle.

La présente invention a donc pour objet une composition cosmétique sous forme d'une laque aérosol pour la fixation des cheveux, contenant dans un solvant, en tant que substance filmogène, un tétrapolymère résultant de la copolymérisation de :

(a) 4 à 6 % en poids d'acide acrylique (AA),
(b) 42 à 52 % en poids de N-vinylpyrrolidone (NVP),
(c) 15 à 25 % en poids de N-tertiobutylacrylamide (Nt BA), et
(d) 20 à 26 % en poids de méthacrylate d'éthyle (MAE), les fonctions acides carboxyliques dudit tétrapolymère étant neutralisées, partiellement ou totalement, à l'aide d'une base minérale ou organique, le gaz propulseur de ladite laque étant au moins un composé non halogéné.

Les très bonnes propriétés des laques aérosols selon l'invention sont dues essentiellement aux choix des monomères du tétrapolymère et en particulier de la N-tertiobutylacrylamide et du méthacrylate d'éthyle et à leurs proportions respectives quine varient que dans une gamme relativement très étroite et critique.

Le taux de neutralisation des fonctions carboxyliques du tétrapolymère est d'au moins 50 % et de préférence compris entre 70 et 100 %.

Parmi les bases minérales susceptibles d'être utilisées on peut notamment citer la soude, la potasse ou l'ammoniaque et parmi les bases organiques les amines primaires et secondaires ou tertiaires, les alcanolamines telles que la triéthanolamine ou la tri-isopropanolamine, les hydroxy-amines tels que l'amino-2-méthyl-2-propanol (AMP) et l'amino-2-méthyl-2-propanediol-1,3 (AMPD).

Le tétrapolymère des laques aérosols selon l'invention doit de préférence avoir un poids moléculaire tel que sa viscosité absolue soit comprise entre 2 et 5 cP (mesurée en solution dans le DMF à 5 % et à 34,6 °C) et de préférence comprise entre 2,3 et 4,5 (m•Pa•s) (cP).

Dans les laques aérosols selon l'invention le tétrapolymère est généralement présent en solution dans un solvant organique à une concentration comprise entre 0,5 et 5 % en poids et de préférence entre 1 et 4 % en poids par rapport au poids total de la composition.

Le solvant est de préférence un alcool inférieur présent à une concentration comprise entre 40 et 60 % en poids et parmi ceux-ci on peut notamment mentionner l'éthanol, l'isopropanol et leurs mélanges. La proportion du gaz propulseur non halogéné est généralement comprise entre 40 et 65 % en poids et de préférence entre 45 et 60 %.

Comme gaz propulseur de la laque aérosol selon l'invention on utilise de préférence un hydrocarbure non halogéné tel que le propane, le n-butane, l'isobutane ou leurs mélanges, en particulier les mélanges de propane-butane dans un rapport de 1:10 à 1:2 ou encore le diméthyléther ou un mélange de ces gaz propulseurs.

Bien entendu les laques aérosols selon l'invention peuvent également contenir d'autres ingrédients conventionnels pour ce type de composition tels que des agents plastifiants, des agents adoucissants, des parfums, des huiles, des lubrifiants, de la lanoline, des silicones, des filtres solaires ainsi que des colorants.

Selon l'invention il est souhaitable que dans la bombe aérosol la pression de vapeur soit comprise entre environ 2,5 et 5 bars à 25°C.

Le tétrapolymère de la laque aérosol selon l'invention peut être obtenu selon les techniques conventionnelles de polymérisation c'est-à-dire en masse, en solution, en suspension, en dispersion ou en émulsion sans que les caractéristiques essentielles du tétrapolymère obtenu en soient affectées.

La réaction de polymérisation peut être initiée à l'aide du catalyseur conventionnel tel que l'azobisisobutyronitrile ou des composés organiques peroxydés comme le peroxyde de lauroyle ou le peroxyde de benzoyle.

En vue de régler le poids moléculaire, il peut être souhaitable d'utiliser des agents de transfert de chaînes tels que des thiols par exemple le mercaptoéthanol ou le dodécanethiol ou encore des accélérateurs qui augmentent la vitesse de réaction.

Selon un mode de réalisation préféré, la polymérisation est effectuée en solution, de préférence dans l'éthanol et en fin de polymérisation, le tétrapolymère est isolé par précipitation notamment dans l'éther de pétrole.

Selon cette forme de réalisation la polymérisation en solution est généralement conduite à une température comprise entre 40 et 90°C et de préférence entre 45 et 80°C.

On va maintenant donner à titre d'illustration et sans aucun caractère limitatif plusieurs exemples de préparation des tétrapolymères ainsi que plusieurs exemples de laques aérosols les contenant.

EXEMPLE 1 : Préparation du tétrapolymère "acide acrylique (AA) 5%/N-vinylpyrrolidone (NVP) 45%/N-tertiobutylacrylamide (Nt BA) 25 %/méthacrylate d'éthyle (MAE) 25 %".

Dans un ballon, sous agitation et sous azote, on introduit 5 g d'acide acrylique, 45 g de N-vinylpyrrolidone, 25 g de N-tertiobutylacrylamide et 25 g de méthacrylate d'éthyle.

On ajoute alors 100 g d'éthanol absolu et 0,75 g d'azobisisobutyronitrile en tant que catalyseur.

Le mélange est chauffé depuis la température ambiante jusqu'à 78°C. La montée de la température est effectuée en 30 min. Cette température est maintenue ensuite pendant environ 18 heures.

On ramène alors le milieu, sous agitation, à température ambiante et on ajoute 600 g d'acétate d'éthyle.

La solution diluée est ensuite précipitée dans 7 litres d'éther de pétrole. Après purification et séchage en étuve sous vide à 60°C, le tétrapolymère attendu est obtenu avec un rendement de 98 %.

Viscosité absolue ( ) : 3,27 (m•Pa•s) (cP) (mesurée en solution à 5 % dans le DMF et à 34,6°C)

Indice d'acide (IA) : 48,9

Selon le même mode opératoire que décrit ci-dessus on a également préparé les tétrapolymères rassemblés dans le tableau I suivant :

TABLEAU I

| EXEMPLES | AA % | NVP % | $N_t$ BA % | MAE % | $Rd_t$ % | | IA |
|---|---|---|---|---|---|---|---|
| 2 | 6 | 43 | 25 | 26 | 95 | 3,2 | 48 |
| 3 | 5 | 48 | 22 | 25 | 96 | 3,0 | 37 |
| 4 | 5 | 51 | 19 | 25 | 96 | 3,1 | 41 |
| 5 | 5 | 49 | 24 | 22 | 98 | 2,9 | 38 |
| 6 | 4 | 46 | 25 | 25 | 96 | 3,0 | 29 |
| 7 | 4 | 50 | 21 | 25 | 97 | 3,3 | 30 |

## EXEMPLES DE LAQUES AEROSOLS

### EXEMPLE A :

On prépare une laque aérosol pour cheveux en conditionnant dans un récipient aérosol 2 g du tétrapolymère préparé selon l'exemple 2, neutralisé à 100 % (d'après l'indice d'acide) par de l'AMP, et 45 g d'éthanol anhydre.

On introduit ensuite selon les techniques conventionnelles 53 g d'un mélange d'hydrocarbures constitué d'isobutane : 55, de butane : 35 et de propane : 10 et on procède à la fixation de la valve et à la fermeture hermétique du récipient (pression : 4 bars).

Par application de la laque sur des cheveux naturels ou des cheveux sensibilisés, on constate un bon pouvoir laquant, une absence de poissage au toucher au moment de l'application, les cheveux se démêlant facilement et ne provoquant qu'un léger effet de poudrage.

Après plusieurs jours sans shampooing ces propriétés sont maintenues. On note en effet un bon démêlage, une absence de poissage et un état des cheveux excellent.

Après shampooing la substance filmogène est parfaitement éliminée.

## ETUDES COMPARATIVES

Afin de mettre en évidence l'importance non seulement du choix des monomères du tétrapolymère mais également leurs proportions relatives sur les propriétés des laques aérosols selon l'invention, on a également préparé les polymères de référence 8 à 12 rassemblés dans le tableau II suivant :

TABLEAU II

| EXEMPLES DE REFERENCE | AA % | NVP % | $N_t$ BA % | MAE % | $Rd_t$ % | | IA |
|---|---|---|---|---|---|---|---|
| 8 | 3 | 30 | 22 | 45 | 99 | 3,33 | 24 |
| 9 | 4 | 36 | 25 | 35 | 99 | 2,75 | 33,6 |
| 10 | 10 | 60 | 20 | 10 | 94 | 3,5 | 77 |
| 11 | 3 | 60 | 20 | 17 | 98 | 3,3 | 24 |
| 12 | 9,2 | 20 | 43,2 | 27,6 | 97 | 3,3 | 69 |

On a alors soumis l'ensemble des tétrapolymères à différents tests qui ont été réalisés dans les conditions suivantes :

### 1. Test de compatibilité aux hydrocarbures:

Dans un flacon aérosol en verre de 100 ml on introduit 6 g de solution alcoolique de polymère à 7,5 % d'extrait sec en polymère neutralisé à 100 % par l'AMP. Après avoir serti le flacon on introduit sous pression le mélange d'hydrocarbures suivant : butane 55, propane 10 et isobutane 35 jusqu'à la limite de limpidité de la solution contenue dans le flacon aérosol. On note le poids x en grammes de mélange d'hydrocarbures introduit provoquant le début d'apparition au trouble. La compatibilité limite aux hydrocarbures est exprimée par l'expression :

$$\frac{6}{6 + x} \cdot 100$$

2. Test de tenue au froid de l'aérosol :

On introduit dans un flacon aérosol pour une phase alcoolique de 45 % : 18 g de solution alcoolique 6,67 % en polymère neutralisé à 100 % par l'AMP. On ajoute 22 g du mélange d'hydrocarbures suivant : butane 55, propane 10 et isobutane 35.

On introduit alors l'aérosol dans un bain thermostaté en refroidissant progressivement la température, de la température ambiante à -25°C, par paliers de 5°C avec un temps de contact de 15 min. pour chaque température. On note la température provoquant l'apparition du trouble.

3. Test de solubilité au shampooing :

Dans un cristallisoir de 55 mm de diamètre, on introduit 2 g de solution alcoolique à 10 % en poids de polymère neutralisé à 100 % par l'AMP, puis l'on procède au séchage du film 24 heures à température ambiante. Sur le film sec, on verse 20 g d'une solution aqueuse à 1,4 % de lauryléther sulfate de sodium.

On note le temps nécessaire à la dissolution du film sous agitation magnétique et la liquidité de la solution obtenue.

4. Test de poissage :

Dans un moule revêtu de "Teflon" de surface de base de 25 cm², on introduit 5 g d'une solution alcoolique à 10 % en poids de polymère neutralisé à 100 % par l'AMP, puis le solvant est évaporé 24 heures à température ambiante et le film est décollé du moule. Le poissage sur les deux faces du film est estimé au toucher par un panel de 5 personnes selon la notation suivante :

0 :    absence de poissage
1 :    poissage très léger
2 :    poissage léger
3 :    poissage moyen
4 :    poissage important
5 :    poissage très important

Le poissage sur film est noté après séchage du film 24 heures dans une atmosphère à 45 % d'humidité relative (HR) et après séchage d'un autre film 24 heures dans une atmosphère à 60 % d'humidité relative (HR).

5. Etude sur mèches

On prépare une laque aérosol pour cheveux en conditionnant dans un récipient aérosol 2g de tétrapolymère, neutralisé à 100 % (d'après l'indice d'acide) par l'AMP et 43g d'éthanol anhydre. On introduit ensuite selon les techniques conventionnelles 55g d'un mélange propulseur constitué de butane 55, propane 10 et isobutane 35 et on procède à la fixation de la valve et à la fermeture hermétique du récipient (pression = 4 bars).

La laque est pulvérisée sur des mèches de cheveux naturels.
- les propriétés sont notées par un panel de 5 personnes après une première vaporisation, après 3 vaporisations et après 5 vaporisations.

Les valeurs données correspondent à une moyenne générale sur les estimations des 5 vaporisations en particulier pour :
. pouvoir laquant
. poissage des cheveux au toucher après application.

Les tableaux III et IV rassemblent les résultats pour les exemples cités :

TABLEAU III

| EXEMPLES | test de compatibilité aux hydrocarbures | test de tenue au froid de l'aérosol | test de solubilité au shampooing anionique | test de poissage poissage sur film | |
|---|---|---|---|---|---|
| | | | | à 45%HR | à 60%HR |
| 1 | 39,2 | < -25°C | limpide 4'20 | 0 | 2 |
| 2 | 39,7 | -20°C | limpide 3' | 2 | 2 |
| 3 | 38,7 | < -25°C | limpide 6'14 | 0 | 2 |
| 4 | 39,5 | -25°C | limpide 2'30 | 0 | 2 |
| 5 | 37,3 | < -25°C | limpide 3'40 | 1 | 2 |
| 6 | 36,4 | < -25°C | limpide 6'30 | 0 | 2 |
| 7 | 37,3 | < -25°C | limpide 4' | 0 | 2 |
| 8 | 42,5 | + 5°C | insoluble | 0 | 2 |
| 9 | 36 | -20°C | très trouble 4'30 | 0 | 2 |
| 10 | 47,2 | impossible | limpide 1'30 | 2 | 4 |
| 11 | 35 | < -25°C | limpide 2'19 | 0 | 4 |
| 12 | 35,5 | < -25°C | limpide 2'45 | 0 | 4 |

6. Etudes sur mèches = moyennes des résultats sur 5 vaporisations

a) Poissage au toucher :

TABLEAU IV

| EXEMPLES | NOTES |
|---|---|
| 1 | 0 |
| 2 | 1 |
| 3 | 0 |
| 4 | 0 |
| 5 | 0 |
| 6 | 0 |
| 7 | 0 |
| 8 | 0 |
| 9 | 0 |
| 10 | 4 |
| 11 | 4 |
| 12 | 4 |

b. Pouvoir laquant :

Il est correct pour tous les exemples.

Comme on peut le constater d'après les tableaux III et IV, seuls les tétrapolymères selon l'invention, c'est-à-dire selon les exemples 1 à 7, présentent les propriétés nécessaires à une utilisation pour laque aérosol.

- Les exemples 8 et 9 ne peuvent convenir parce qu'ils ne présentent pas une solubilité suffisante aux shampooings anioniques.
- Les exemples 10, 11 et 12 ne peuvent également convenir parce qu'ils donnent un poissage au toucher trop important à 60 % d'humidité relative, confirmé par le test de poissage sur mèches.

**Revendications**

**1.** Composition cosmétique sous forme d'une laque aérosol pour la fixation les cheveux, caractérisée par le fait qu'elle contient dans un solvant, en tant que substance filmogène un tétrapolymère résultant de la copolymérisation de :

(a) 4 à 6 % en poids d'acide acrylique (AA),

(b) 42 à 52 % en poids de N-vinylpyrrolidone (NVP),

(c) 15 à 25 % en poids de N-tertiobutylacrylamide (Nt BA), et

(d) 20 à 26 % en poids de méthacrylate d'éthyle (MAE), les fonctions acides carboxyliques dudit tétrapolymère étant neutralisées, partiellement ou totalement à l'aide d'une base minérale ou organique, le gaz propulseur de ladite laque étant au moins un composé non halogéné.

2. Composition selon la revendication 1, caractérisée par le fait que le tétrapolymère présente une viscosité absolue comprise entre 2 et 5 (m•Pa•s) (cP) (mesurée en solution dans le DMF à 5 % et à 34,6°C).

3. Composition cosmétique selon la revendication 1 ou 2, caractérisée par le fait que les fonctions acides carboxyliques sont neutralisées à un taux d'au moins 50 % et de préférence compris entre 70 et 100 %.

4. Composition cosmétique selon l'une quelconque des revendications 1 à 3, caractérisée par le fait que les fonctions acide carboxylique sort neutralisées à l'aide d'une base minérale choisie parmi la soude, la potasse et l'ammoniaque.

5. Composition cosmétique selon l'une quelconque des revendications 1 à 3, caractérisée par le fait que les fonctions acides carboxyliques sont neutralisées à l'aide d'une base organique choisie parmi la triéthanolamine, la tri-isopropanolamine, l'amino-2-méthyl-2-propanol et l'amino-2-méthyl-2-propanediol-1,3.

6. Composition cosmétique selon l'une quelconque des revendications précédentes caractérisée par le fait que le tétrapolymère est présent à une concentration comprise entre 0,5 et 5 % et de préférence entre 1 et 4 % en poids par rapport au poids total de la composition.

7. Composition cosmétique selon l'une quelconque des revendications précédentes, caractérisée par le fait que le gaz propulseur est présent en une proportion comprise entre 40 et 65 % en poids.

8. Composition cosmétique selon l'un quelconque des revendications précédentes, caractérisée par le fait que le gaz propulseur non halogéné est un hydrocarbure pris dans le groupe constitué par le propane, le n-butane, l'isobutane et leurs mélanges.

9. Composition cosmétique selon la revendication 8, caractérisée par le fait que le gaz propulseur non halogéné est un mélange propane-butane dans un rapport 1:10 à 1:2.

10. Composition cosmétique selon l'une quelconque des revendications 1 à 8, caractérisée par le fait que le gaz propulseur non halogéné est le diméthyléther ou son mélange avec au moins un hydrocarbure non halogéné.

11. Composition cosmétique selon l'une quelconque des revendications précédentes, caractérisée par le fait que le solvant est présent en une proportion comprise entre 40 et 60 % en poids par rapport au poids total de la composition.

12. Composition cosmétique selon l'une quelconque des revendications précédentes, caractérisée par le fait que le solvant est l'éthanol, l'isopropanol ou leurs mélanges.

**Claims**

1. Cosmetic composition in the form of an aerosol lacquer for setting hair, characterized in that it contains, in a solvent, as film-forming substance, a tetrapolymer resulting from the copolymerization of:

(a) 4 to 6 % by weight of acrylic acid (AA),

(b) 42 to 52 % by weight of N-vinylpyrrolidone (NVP),

(c) 15 to 25 % by weight of N-tert-butylacrylamide (NEBA), and

(d) 20 to 26 % by weight of ethyl methacrylate (EMA), the carboxylic acid functional groups of the said tetrapolymer being partially or completely neutralized with the aid of an inorganic or organic base, the propellent gas for the said lacquer being at least one nonhalogenated compound.

2. Cosmetic composition according to Claim 1, characterized in that the tetrapolymer has an absolute viscosity of between 2 and 5 mPa s (cP) (measured in solution in DMF at a concentration of 5 % and at 34.6 ° C).

3. Cosmetic composition according to Claim 1 or 2, characterized in that the carboxylic acid functional groups are neutralized in a proportion of at least 50 % and preferably of between 70 and 100 %.

4. Composition according to any one of Claims 1 to 3, characterized in that the carboxylic acid functional groups are neutralized with the aid of an inorganic base chosen from sodium hydroxide, potassium hydroxide and aqueous ammonia.

5. Cosmetic composition according to any one of Claims 1 to 3, characterized in that the carboxylic acid functional groups are neutralized with the aid of an organic base chosen from triethanolamine, triisopropanolamine, 2-amino-2-methylpropanol and 2-amino-2-methyl-1,3-propanediol.

6. Cosmetic composition according to any one of the preceding claims, characterized in that the tetrapolymer is present in a concentration of between 0.5 and 5 % and preferably between 1 and 4 % by weight relative to the total weight of the composition.

7. Cosmetic composition according to any one of the preceding claims, characterized in that the propellent gas is present in a proportion of between 40 and 65 % by weight.

8. Cosmetic composition according to any one of the preceding claims, characterized in that the nonhalogenated propellent gas is a nonhalogenated hydrocarbon taken from the group consisting of propane, n -butane, isobutane and mixtures thereof.

9. Cosmetic composition according to Claim 8, characterized in that the nonhalogenated propellent gas is a propane-butane mixture in a ratio of 1:10 to 1:2.

10. Cosmetic composition according to any one of Claims 1 to 8, characterized in that the nonhalogenated propellent gas is dimethyl ether or its mixture with at least one nonhalogenated hydrocarbon.

11. Cosmetic composition according to any one of the preceding claims, characterized in that the solvent is present in a proportion of between 40 and 60 % by weight relative to the total weight of the composition.

12. Cosmetic composition according to any one of the preceding claims, characterized in that the solvent is ethanol, isopropanol or mixtures thereof.

**Patentansprüche**

1. Kosmetische Zubereitung in Form eines Lack-Aerosols zum Festigen der Haare, dadurch gekennzeichnet, daß sie in einem Lösungsmittel als filmbildende Substanz ein Tetrapolymer enthält, welches durch Copolymerisieren von
   (a) 4 bis 6 Gewichtsprozent Acrylsäure (AS),
   (b) 42 bis 52 Gewichtsprozent N-Vinylpyrrolidon (NVP),
   (c) 15 bis 25 Gewichtsprozent N-tert-Butyl-acrylamid (N-t-BA) sowie
   (d) 20 bis 26 Gewichtsprozent Ethylmethacrylat (EMA)
   dargestellt wird, wobei die Carbonsäurefunktionen des vorstehenden Tetrapolymers in partiell oder völlig neutralisierter Form vorliegen, die Neutralisation mit Hilfe einer mineralischen oder organischen Base erfolgt ist und das Treibgas des Haarlacks aus mindestens einer nichthalogenierten Verbindung besteht.

2. Zubereitung gemäß Anspruch 1, dadurch gekennzeichnet, daß das Tetrapolymer eine absolute Viskosität im Bereich zwischen 2 und 5 m Pa s (cP) aufweist, wobei die Messung in 5 %iger DMF-Lösung bei 34,6 ° C vorgenommen wurde.

**3.** Kosmetische Zubereitung gemäß Anspruch 1 oder 2, dadurch gekennzeichnet, daß die Carbonsäurefunktionen in einem Ausmaß von mindestens 50% und vorzugsweise in einem Ausmaß, der im Bereich zwischen 70 und 100% liegt, neutralisiert sind.

**4.** Kosmetische Zubereitung gemäß einem der Ansprüche 1 bis 3, dadurch gekennzeichnet, daß die Carbonsäurefunktionen mit Hilfe einer Mineralbase neutralisiert worden sind, welche aus Natriumcarbonat, Kaliumcarbonat und Ammoniaklösung ausgewählt ist.

**5.** Kosmetische Zubereitung gemäß einem der Ansprüche 1 bis 3, dadurch gekennzeichnet, daß die Carbonsäurefunktionen mit Hilfe einer organischen Base neutralisiert worden sind, welche unter Triethanolamin, Triisopropanolamin, 2-Amino-2-methyl-propanol und 2-Amino-2-methyl-propan-1,3-diol ausgewählt worden ist.

**6.** Kosmetische Zubereitung gemäß einem der vorstehenden Ansprüche, dadurch gekennzeichnet, daß das Tetrapolymer in einer Konzentration zwischen 0,5 und 5 Gewichtsprozent, vorzugsweise zwischen 1 und 4 Gewichtsprozent in Bezug auf das Gesamtgewicht der Zubereitung vorliegt.

**7.** Kosmetische Zubereitun gemäß einem der vorstehenden Ansprüche, dadurch gekennzeichnet, daß das Treibgas in einem Mengenanteil zwischen 40 und 65 Gewichtsprozent vorliegt.

**8.** Kosmetische Zubereitung gemäß einem der vorstehenden Ansprüche, dadurch gekennzeichnet, daß das nichthalogenierte Treibgas ein Kohlenwasserstoff ist, welcher aus der aus Propan, n-Butan, Isobutan und deren Gemischen bestehenden Gruppe ausgewählt ist.

**9.** Kosmetische Zubereitung gemäß Anspruch 8, dadurch gekennzeichnet, daß das nichthalogenierte Treibgas eine Mischung aus Propan/Butan im Verhältnis von 1 : 10 bis 1 : 2 darstellt.

**10.** Kosmetische Zubereitung gemäß einem der Ansprüche 1 bis 8, dadurch gekennzeichnet, daß das nichthalogenierte Treibgas aus Dimethylether oder dessen Mischung mit mindestens einem nichthalogeniertem Kohlenwasserstoff besteht.

**11.** Kosmetische Zubereitung gemäß einem der vorstehenden Ansprüche, dadurch gekennzeichnet, daß das Lösungsmittel in einem Mengenverhältnis zwischen 40 und 60 Gewichtsprozent in Bezug auf das Gesamtgewicht der Zubereitung vorhanden ist.

**12.** Kosmetische Zubereitung gemäß einem der vorstehenden Ansprüche, dadurch gekennzeichnet, daß das Lösungsmittel aus Ethanol, Isopropanol oder deren Gemischen besteht.